# EUROPEAN PATENT APPLICATION

(11) **EP 3 593 849 A1**
(43) Date of publication of application: **15.01.2020**
(21) Application number: 18183079.5
(22) Date of filing: 12.07.2018
(51) Int. Cl.: A61M 25/00

(54) **CATHETER WITH COLLAPSIBLE OUTER LUMINA**

(71) Applicant: Medtentia International Ltd Oy, 02600 Espoo (FI)
(72) Inventor: Keränen, Olli, 23741 Bjärred (SE)
(74) Representative: Berggren Oy, Helsinki & Oulu

(57) **Abstract**

A multilumen catheter (100) for vascular applications comprises posterior (100A) and distal (100B) ends and at least one first (101) and second (102, 103) lumens extending between the posterior (100A) and distal (100B) ends of the catheter. Each lumen (101, 102, 103) comprises an outer wall structure (105) and are separated from each other by an inner wall structure (106), wherein said outer wall and inner wall structures form said lumens. The catheter has an activated state and inactivated state, wherein in said inactivated state the diameter or volume of the lumen determined by said outer (105) wall and inner wall (106) structures is smaller than the diameter or volume of the lumen in said activated state.

## Description

### TECHNICAL FIELD OF THE INVENTION

The invention relates to a catheter and in particularly to a catheter for cardiovascular applications. In addition the invention relates to a manufacturing method for manufacturing the catheter.

### BACKGROUND OF THE INVENTION

Different kinds of catheters are known from prior art, also for cardiovascular applications. The known catheters have typically a lumen extending between posterior and distal ends of the catheter. However, problems arise when two or more objects are tried to deliver though the lumen of the catheter, namely number of guide wires of the objects or other members delivered through the lumen at the same will tangle with each other very easily. In addition, the insertion of the catheter is often dramatic operation for organs, especially when the catheter with large diameter is inserted.

### SUMMARY OF THE INVENTION

An object of the invention is to alleviate and eliminate the problems relating the known prior art. Especially the object of the invention is to provide a catheter, by which number of object can be delivered or guided through the catheter without a risk that the object or the guide wires or the like would tangle with each other in the catheter. In addition the object of the invention is to provide a catheter, which stress the organs minimally when inserted and also during an operation.

The object of the invention can be achieved by the features of independent claims.

The invention relates to a catheter according to claim 1. In addition the invention relates to a manufacturing method for manufacturing a catheter according to claim 21.

According to an embodiment of the invention a catheter comprises at least first and second lumens extending between posterior and distal ends of the catheter. Each lumen comprises an outer wall structure and are separated from each other by an inner wall structure so that said outer wall and inner wall structures form the lumens. By the separating walls it can be ensured that the objects or guide members or other means or fluids delivered through the catheter are not got tangled or mixed with each other during introducing or delivering. In addition the using of more than one lumen offers also other advantages, namely the more than one objects can be introduced at the same time without possibility that the objects touch or tangle or mix each other. For example different drugs can be delivered through different lumens, or one lumen can be used for delivering e.g. flushing agent, a second lumen can be used for rinsing, and still a third or any additional lumen can be used for delivering operating devices or implants or other object to the target without mixing the objects of first, second and third lumens with each other.

Advantageously at least one of the first and second lumens has an activated state and inactivated state. In the inactivated state the diameter (*d₂*) or volume (*V₂*) of the lumen determined by said outer wall and inner wall structures is smaller than the diameter (*d₁*) or volume (*V₁*) in said activated state. The inactivated state can be achieved when the outer wall structure of at least one of said first and second lumens is compressible and advantageously also flexible so that the outer walls, as well as also inner walls forming the lumens can be compressed in order to provide the inactivated state. Alternatively, or in addition to, the outer and/or inner wall structure of at least one of said first and second lumens is foldable so that when the wall structure is folded, the catheter has its inactivated state, and when the wall structure of at least one lumen is at least partly unfolded, the catheter (or at least the lumen in question) is in the activated state.

The wall structures of the lumens are advantageously arranged so that at least one of the first and second lumens is in the inactivated state when no external or internal force is applied, whereupon the wall structures defining said lumen in question is compressed and/or folded or pushed smaller in any other way. Advantageously the inactivated state is taken as a default and thereby the diameter or volume of the catheter is minimized without any external efforts.

The catheter with the inactivated and activated states offers clear advantages, namely the catheter can be inserted in the inactivated state, when the diameter or volume is small and advantageously the smallest possible (the outer and possible also inner wall are compressed and/or folded), whereupon the insertion can be done so that only minimal damage or stress is incurred to the organs. When the objects are delivered through the one or more lumens, only the diameter or volume of the lumen in question is increased, whereupon all the other lumens still rest in the inactivated state with minimum diameter. In addition it is to be noted that the lumen used is in the activated state only temporary so only the time when the object is delivered, after which the lumen will take its inactivated state again.

It is to be noted that at least one of said lumen is configured to change the state from the inactivated state to the activated state when an object is introduced through said lumen and then change or collapse (for example folding) the state back to or at least towards the inactivated state when the object has passed through said lumen. Especially it should be noted that the subsequent inactivated state of the lumen after collapsing or shrinking might not be exactly the same as the initial inactivated state before the activation, so for example the diameter or volume of the lumen might be slightly greater or smaller after collapsing or shrinking back to or thus towards the subsequent inactivated state as was the diameter or volume of the lumen in question in the initial inactivated state. This is because the transformation process of the lumens might not be absolutely and completely reversible process.

According to an example the collapsing or shrinking of at least one lumen can be assisted by a vacuum providing device, such as syringe, to provide a vacuum into the lumen or at least decreasing the pressure to the lumen slightly. For example the lumen may be arranged to receive the syringe or the like by which the lumen can be aspired to empty, which is arranged to cause the lumen in question to collapse or shrink back to or towards the inactivated state.

The delivering of the object takes some volume of the lumen (so increases the diameter or volume temporary). In some cases the delivering may take also a certain volume from the other adjacent lumen(s) also, especially when the adjacent lumen(s) is(are) empty, but after delivering the lumens (also adjacent lumen(s) will change the state back to or at least towards the inactivated state.

In addition, the catheter may also comprise a guiding lumen, which extends between the posterior and distal ends of the catheter, and through which a guide member can be introduced to guide the catheter during operations. The guide member may be wire or other member, which can be operated by an operator from the posterior end and thereby guide the distal end of the catheter for example to a right position. The guiding lumen has thus advantageously non-compressible wall structures. However, the guiding lumen is advantageously flexible and bendable, in particularly the guiding lumen is configured to be bend in its distal end. When the all other lumens are coupled with the guiding lumen (advantageously via the wall structures of them), the all other lumens will follow the guiding lumen when the guiding lumen is bend.

According to an example the guide member, such as a guide wire, comprises a sharp distal end so that it can penetrate though the tissue.

According to an embodiment the outer wall structure of the all first and second lumens may be physically a common outer wall structure for the first and second lumens and made of the same material. This makes the manufacturing of the catheter easy and fast, when there is no need to manufacture separate walls and after this to connect the wall structures to each other. It is to be, however, noted that even if the lumens may have the common outer wall structure, the outer wall can still be foldable and/or compressible. Depending on the application the inner wall structures (or some of them) may also be common structures or alternatively separated structures.

According to an embodiment the inner and/or outer wall structure of the first lumen comprises first material and the inner and/or outer wall structure of the second lumen comprises second material, where elasticity or other material property of said first and second material differs from each other. In this way a certain lumen can be prioritized, for example.

In addition, according to an embodiment the catheter comprises a closing member, such as a valve, for closing and opening at least one lumen. The closing member may be common for more than one lumen or then for a one dedicated lumen. In a closed position the closing member advantageously prevents any backflow or leakage of fluids via the lumen between the posterior and distal ends. In addition it is important to prevent air flowing to the distal portion of the catheter and thereby getting into the heart, for example. During operation this might be important feature for isolating the opening of the distal end of the catheter and thereby the organ to be operated from external environment and unwanted impurity, for example. In addition it is important to avoid to air The closing member can be implemented in many different ways known from the prior art, such as by a valve, flap or ball and it can be operated for example by turning or pressing, for example, but not limiting to those examples only.

Moreover, according to an embodiment the catheter may comprise a radiopaque member. The radiopaque member is advantageously made of or comprises material, such as metal, which can be seen by X-ray or echo. The radiopaque member is advantageously located in the distal end of the catheter. Still, in addition, at least one lumen may also comprise a flushing or rinsing possibility in order to flush or rinse the lumen. As an example only one lumen may have this option, or alternatively at least two or more or even all lumens may have the flushing possibility. This can be implemented for example by a Y-connector for example with a luer lock.

According to an embodiment the first and second lumens may be different in length for example so that the first lumen is shorter and the second lumen. In particularly the distal ends of the first and second lumens may be at different depths or non-aligned, whereupon for example there is much more space left into the distal end area of the catheter. This allows in some cases to use tools or instruments easier in the distal portion of the catheter, such as providing a capturing device for capturing a suture or other fixing means via the second lumen (extending further or deeper than the first lumen) and turn it against the extension line of the first shorter lumen, though which the suture or other fixing means is then delivered.

The embodiments described in this document offer many advantages over the known prior art, such as more than one objects can be introduced at the same time without possibility that the objects touch or tangle with each other during introduction through the catheter. In addition the inactivated and activated states offer clear advantages, namely the catheter can be inserted in the inactivated state very easily and with minimal stress or damages.

Further, when the objects are delivered through the one or more lumens, only the diameter or volume of the lumen in question is increased. Thus the changes of the catheter occurs during delivering of the objects only and according to an embodiment essentially or mainly in a direction perpendicular to the longitudinal axis extending between the proximal and distal ends of the catheter, which is much less stressful for the organs, such as veins or vessels along which the catheter is inserted. This is because the pressure of the increased diameter or volume of the catheter occurs only against the wall of the veins or vessels, and not in the direction of the wall and especially during inserting of the catheter, which would cause friction and in that way easily cause damages to the organs.

The exemplary embodiments presented in this text are not to be interpreted to pose limitations to the applicability of the appended claims. The verb "to comprise" is used in this text as an open limitation that does not exclude the existence of also unrecited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:

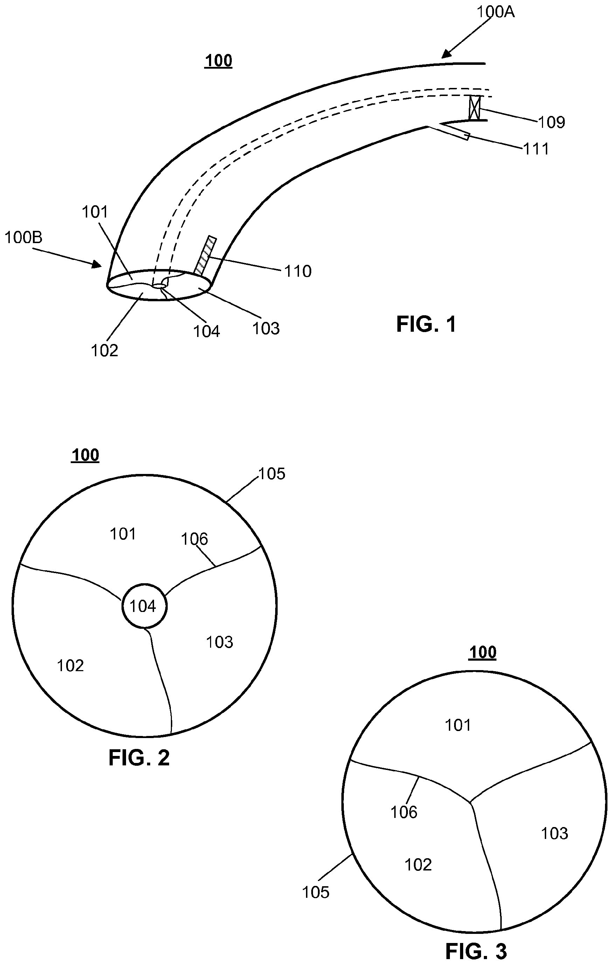

- Figures 1-10: illustrate examples of catheters according to advantageous embodiments of the invention.

### DETAILED DESCRIPTION

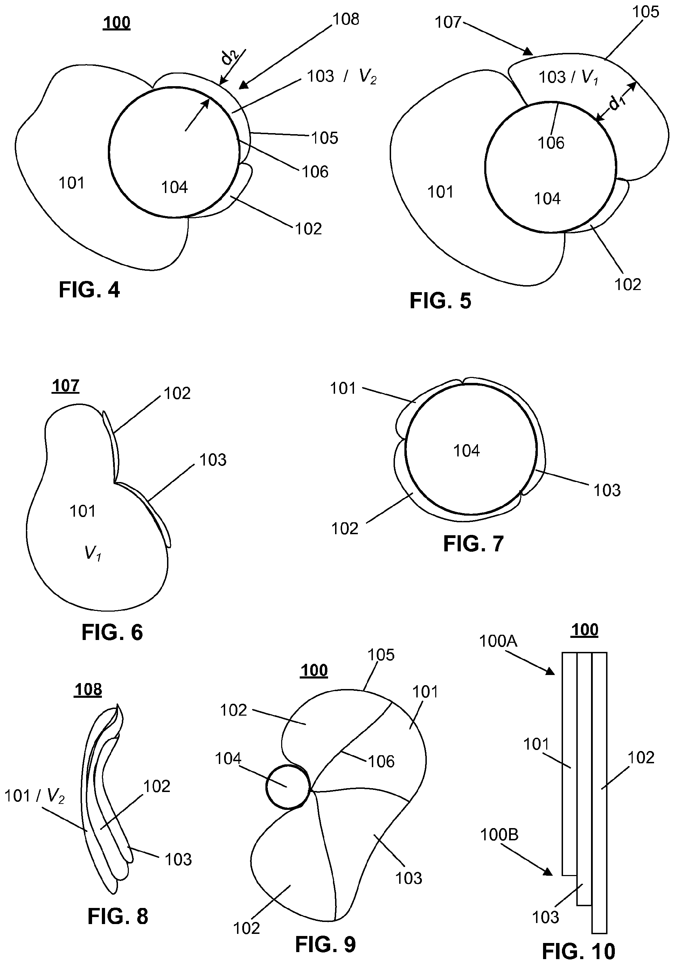

Figures 1-10 illustrate examples of catheters 100 according to advantageous embodiments of the invention, wherein the catheter 100 comprises at least first 101 and second 102, 103 lumens extending between posterior 100A and distal 100B ends of the catheter 100. It is to be noted, that the catheter may have plurality of lumens, as is described e.g. in Figure 9. Each lumen 101, 102, 103 comprises an outer wall structure 105 and are separated from each other by an inner wall structure 106.

As can be seen in Figures 1, 2, 4-5, 7 and 9, the catheter 100 may also comprise a guiding lumen 104, which extends between the posterior 100A and distal 100B ends of the catheter, and through which a guide member (not shown) can be introduced to guide the catheter during operations. The guiding lumen 104 is advantageously flexible and bendable and in particularly it is configured to be bend in its distal end 100B. When the all other lumens 101-103 are coupled with the guiding lumen (advantageously via the wall structures 105, 106 of them), the all other lumens 101-103 will follow the guiding lumen 104 when the guiding lumen 104 is bend or otherwise moved.

However, the separate guiding lumen 104 is an optional and according to an embodiment the catheter 100 can also be implemented only with the at least one first and second lumens 101-103, as is the case in Figure 3, 6 and 8. In these examples one of the first and second lumens 101-103 can be used for guide members, if needed.

Figures 4-6 illustrate different states of the catheter 100. For example in Figure 4 the second lumen 103 is in an inactivated state (108 in Figure 8), wherein the diameter *d₂* or volume *V₂* of the second lumen 103 determined by the outer wall and 105 inner wall 106 structures is smaller than illustrated in Figure 5, where the second lumen 103 is in an activated state (107 in Figure 6) and has greater the diameter *d₁* or volume *V₁*. In Figures 4 and 5 the first lumen 101 is in an activated state, whereas another second lumen 102 is in an inactivated state in both Figures 4 and 5. In Figure 7 all first and second lumens 101, 102, 103 are in the inactivated state.

Figure 6 illustrates a situation where the first lumen 101 is in the activated state 107 with volume *V₁*, and Figure 8 a situation where the first lumen 101 is in the inactivated state 108 with volume *V₂* being smaller than the volume with volume *V₁* in the activated state 107. It is to be noted that the catheter 100 illustrated in Figures 6 and 8 does not have any separate guiding lumen 104, but one of the first or second lumen 101-103 can be used for guide member.

The outer wall structure 105 of the all first and second lumens 101-103 may be physically a common outer wall structure 105 for the first and second lumens 101-103, as is the case for example in Figures 1-3 and 9, or alternatively at least one of the first and second lumens 101-103 may have own outer wall structure 105, as is the case for example in Figures 4-8.

In addition, according to an embodiment the catheter comprises a closing member 109, such as a valve, for closing and opening at least one lumen 101-103. The closing member 109 may locate for example in the posterior 100A end for preventing any backflow or leakage or other unwanted transportation of fluids, including air, via the lumen(s) between the posterior 100A and distal 100B ends when closed.

Still in addition the catheter 100 may comprise a radiopaque member 110, advantageously at least in the distal end 100B of the catheter. Furthermore the catheter 100 may comprise also a y-connectorwith a luer lock 111 or the like in order for allowing flushing or rinsing possibility.

Figure 10 illustrates an exemplary catheter 100, where the distal ends 100B of the first and second lumens 101, 102, 103 are at different depths or non-aligned, whereupon much more space is left into the distal end area 100B of the catheter 100. It is to be noted that the lumens 101, 102, 103 are advantageously of different lengths, but may also be same lengths.

The invention has been explained above with reference to the aforementioned embodiments, and several advantages of the invention have been demonstrated. It is clear that the invention is not only restricted to these embodiments, but comprises all possible embodiments within the spirit and scope of the inventive thought and the following patent claims.

The features recited in dependent claims are mutually freely combinable unless otherwise explicitly stated.

## Claims

1. A catheter (100) for vascular applications, the catheter having posterior (100A) and distal (100B) ends,
wherein the catheter comprises:
- at least first (101) and second (102, 103) lumens extending between the posterior (100A) and distal (100B) ends of the catheter, wherein each lumen (101, 102, 103) comprises an outer wall structure (105) and wherein said lumens are separated from each other by at least one inner wall structure (106), wherein said outer wall and inner wall structures form said lumens.

2. A catheter of claim 1, wherein at least one of said first and second lumens (101-104) comprises an activated state (107) and inactivated state (108), wherein in said inactivated state the diameter (d2) or volume *(V₂*) of the lumen determined by said outer (105) wall and inner wall (106) structures is smaller than the diameter (*d₁*) or volume (*V₁*) in said activated state (107).

3. A catheter of any previous claims, wherein the outer wall structure (105) of at least one of said first and second lumens (101-103) is compressible and flexible so that it is compressed in said inactivated state (108).

4. A catheter of any previous claims 2-3, wherein the outer wall structure (105) of at least one of said first and second lumens (101-103) is foldable so that it is folded in said inactivated state (108) and at least partly unfolded in said activated state (107).

5. A catheter of any previous claims 2-4, wherein at least one of said first and second lumens (101-103) is in said inactivated state (108) when no external or internal force is applied.

6. A catheter of any previous claims, wherein the outer wall structure (105) of the all first and second lumens (101-103) is a common outer wall structure for the first and second lumens (101-103).

7. A catheter of claim 6, wherein the common outer wall structure for the first and second lumens (101-103) is made of the same material.

8. A catheter of any of claims 1-6, wherein the inner and/or outer wall structure of the first lumen comprises first material and the inner and/or outer wall structure of the second lumen comprises second material, where elasticity or other material property of said first and second material differs from each other.

9. A catheter of any previous claims, wherein the catheter comprises a guiding lumen (104) extending between the posterior (100A) and distal (100B) ends of the catheter, through which a guide wire can be introduced to guide the catheter during operations.

10. A catheter of claim 9, wherein the guiding lumen (104) is one of the second lumens (102, 103).

11. A catheter of claim 9, wherein the guiding lumen (104) comprises a non-compressible wall structure.

12. A catheter of any claims 9-11, wherein the wall structure of the guiding lumen (104) is flexible and bendable and steerable, in particularly the guiding lumen (104) is configured to be bend in its distal end (100B) so that the all the other lumens (101-103) follow the bending of the guiding lumen (104).

13. A catheter of claim 9-12, wherein all the first and second lumens (101-103) are arranged around the guiding (104) lumen so that the guiding lumen (104) locates essentially at the center of said catheter (100).

14. A catheter of claim 9-13, wherein the guiding lumen (104) locates essentially at a peripheral region of the catheter.

15. A catheter of any previous claims 2-14, wherein at least one of said lumen (101-104) is configured to change the state from the inactivated state (108) to the activated state (107) when an object is introduced through the one lumen (101-104) and then change or collapse the state back to or towards the inactivated state (108) when the object has passed through the one lumen (101-104).

16. A catheter of any previous claims, wherein the first lumen (101) has a first maximum diameter or volume and the second lumen (102, 103) a second maximum diameter or volume in an activated state, where the first maximum diameter or volume differs from said second maximum diameter or volume.

17. A catheter of any previous claims, wherein the catheter comprises a closing device, such as a valve (109) in the proximal end (100A) for closing at least one lumen (101-103) and thereby preventing backflow or leakage or other transportation of fluids, including air, via the lumen between the posterior (100A) and distal (100B) ends.

18. A catheter of any previous claims, wherein the catheter comprises a radiopaque member (110) in the distal end (100B).

19. A catheter of any previous claims, wherein at least one lumen (101-104) comprises a flushing possibility in order to flush the lumen, such as a y-connector with a luer lock (111).

20. A catheter of any previous claims, wherein the first lumen has a first length and the second lumen has a second length, where said first and second lengths differ from each other and/or wherein the distal ends of the first and second lumens are at the different depths or non-aligned.

21. A manufacturing method for manufacturing a catheter (100) according to any of previous claims for vascular applications, where the catheter comprises posterior (100A) and distal (100B) ends,
wherein manufacturing method of the catheter comprises:
- providing at least one outer wall structure (105) defining an outer perimeter of the catheter between the posterior (100A) and distal (100B), and at least one inner wall structure (106) for defining at least one first (101) and second (102, 103) lumens extending between the posterior (100A) and distal (100B) ends of the catheter so that said at least one first (101) and second (102, 103) lumens are separated from each other by said inner wall structure (106) and limited by the outer wall structure (105).
